# EUROPEAN PATENT SPECIFICATION

(11) **EP 2 804 534 B1**
(45) Date of publication and mention of the grant of the patent: **06.05.2020**
(21) Application number: 13700291.1
(22) Date of filing: 11.01.2013
(51) Int. Cl.: A61B 10/02, A61B 17/00

(54) **DEVICE FOR TAKING AT LEAST ONE SAMPLE OF TISSUE**
VORRICHTUNG ZUR ENTNAHME MINDESTENS EINER PROBE AUS EINEM GEWEBE
DISPOSITIF POUR LE PRÉLÈVEMENT D'AU MOINS UN ÉCHANTILLON DE TISSU

(30) Priority: 16.01.2012 EP 12290018
(43) Date of publication of application: 26.11.2014
(73) Proprietor: Coloplast A/S, 3050 Humblebæk (DK)
(72) Inventor: CALLEDE, David, F-24200 Sarlat la Caneda (FR); PIVARD, Laurent, F-01590 Dortan (FR); PINAUD, Denis, F-74550 Draillant (FR); TEPPE, Fabrice, F-01100 Oyonnax (FR); MOINE, Adrien, F-68190 Ensisheim (FR)
(86) International application number: PCT/EP2013/050454
(87) International publication number: WO 2013/107691

(56) References cited:
- WO-A1-99/15079
- WO-A1-03/077767
- WO-A2-2006/081556
- US-A- 5 538 010
- US-A- 5 842 999
- US-A1- 2005 080 355
- US-A1- 2011 137 202

## Description

### TECHNICAL FIELD

The present invention relates to a device for taking at least one sample of soft tissue from an organ, said device comprising a body and a needle formed by a stylet and a cannula coaxial with said stylet, said device comprising a mechanism for arming the needle, designed for sequentially moving the cannula and then the stylet from a rest position wherein the stylet and the cannula are extended towards the outside of the body, to a shooting position wherein the stylet and the cannula are retracted towards the rear of the body, and a triggering mechanism designed to release the stylet then the cannula and to allow their displacement from the shooting position to the rest position, the cannula being coupled kinematically to a cannula slider comprising at least one retaining element for maintaining the cannula slider in a shooting position, the stylet being coupled kinematically to a stylet slider comprising at least one retaining element for maintaining the stylet slider in a shooting position and means for unlocking the cannula slider.

### BACKGROUND ART

Nowadays, there are several devices for taking samples of soft tissue, these devices being generally used to extract, in a minimally invasive way, a sample of an organ from a human or an animal for analysis purpose. This extraction operation is generally known as biopsy and the used device is known as a biopsy gun.

Such a sampling device comprises in particular a sampling needle formed by a cannula and a stylet, an arming mechanism placed on a body and a trigger also placed on the body of the device.

The arming mechanism is used to partially retract the needle towards the inside of the body of the device, the device is placed near the organ from which one wishes to take a sample, then the trigger is pressed so that the needle can penetrate into the organ. The needle being formed by a stylet and by a cannula, the stylet penetrates into the organ, the cannula then covers the stylet. This stylet comprises at least one notch receiving the tissue to be taken. When the cannula covers the stylet, the tissue sample is trapped in the notch and is cut. The unit is withdrawn so that the sample(s) arranged between the stylet and the cannula can be taken. An example of application of such a device is taking tissues of the prostate.

The arming of the needle is generally achieved in two phases, namely the arming of the cannula in a first phase and the arming of the stylet in a second phase.

During sampling of tissues, it is frequent that the person who carries out the sampling has only one free hand, the other hand being used to hold other medical devices such as for instance an echographic probe. In this case, it is important to be able to manipulate the sampling device with one single hand. The manipulation implies here the arming of the cannula, the arming of the stylet and the release of the shot allowing for the sample to be taken.

Among the existing devices, which enable manipulation with one single hand, one of them is described in the US patent 7,153,275. This device is perfectly functional in most cases. However, problems may occur in certain circumstances. These problems can come from the fact that the stylet and the cannula are not perfectly aligned and that the stylet does not slide in a totally optimal way in the cannula. Indeed, an optimal sliding motion involves particularly tight manufacturing tolerances for the realisation of the parts of the biopsy gun. These tolerances can sometimes be difficult to maintain on parts made from plastic. This can lead to jamming of the needle, sometimes even to deformation of the latter. This has also as a consequence the reduction of the number of shots that it is possible to carry out with a device.

In order to minimise the problems linked to the jamming of the stylet in the cannula, a relatively powerful spring is foreseen for the cannula in order to propel the latter in an effective way. This has the drawback that a greater force is necessary to arm the device, which is unpleasant for the user. Despite such a spring, the needle may jam and bend so that the device becomes unusable. US200510080355 discloses a needle set for a biopsy device. The needle set includes an outer member, a cylinder lumen within said outer member, an inner member having a cannula and an inner lumen. The inner member is slidably disposed within the cylinder lumen. The biopsy device also includes a cylinder seal member that is disposed within the cylinder lumen and a cannula seal member attached to the outer surface of the cannula. The cylinder seal member and the cannula seal member define a vacuum chamber therebetween.

US5538010 discloses a biopsy needle device including a needle assembly sequentially driven by a spring loaded drive mechanism.

US5842999 discloses an automated tissue sampling device which permits its needles to be cocked either simultaneously or sequentially and can be cocked with only one hand.

This invention proposes to realize a tissue sampling device which has the advantages of the devices of the prior art i.e. it is possible to use this device with one hand. However, this device does not have the drawbacks of the systems of the prior art. Thus, the risk of jamming of the needle, as well as the risk of breakage or deformation, is strongly reduced or even eliminated.

Furthermore, and especially in implementations of the invention wherein the sampling device may be a single-use sampling device, particularly the risk of jamming of the needle and/or the cannula individually or in relation to each other is reduced or eliminated. This is at least partly because the sampling device, and particularly the movable parts thereof e.g. the needle and the cannula, is then assembled correctly during manufacture leaving no risks of a user putting the parts together in the wrong manner as could very well be the case with re-useable sampling devices. In addition, a single-use device is also significantly less prone to risks of contamination, e.g. by bacteria on a user's hands.

Moreover, as a single-use sampling device may enable production tolerances different from those of a re-useable sampling device, it is in most cases less costly to manufacture than such re-useable sampling devices. Thereby, the improved security mechanisms against unintentional firing of the sampling device according to the different implementations of the invention may be particularly, but not exclusively, suitable for single-use sampling devices in order to meet any potential risks due to such different production tolerances as mentioned above.

### DISCLOSURE OF THE INVENTION

The object of the invention is fulfilled by a sampling device as defined in the appended claims.

According to the present invention, the device for taking samples can easily be manipulated with one hand. For this scope, it comprises a body having an essentially cylindrical shape that can be easily held. It also comprises a sliding arming button, which is positioned on the body so that this button can be easily moved using one finger. This arming button is connected to an arming mechanism, which has two different functions. In a first phase, the displacement of the arming button has the effect of moving the cannula towards the back of the body. When this displacement has been achieved till a certain position, the arming button is released, allowing it to go back to its initial position. When it is operated again, the arming button has another function with respect to the previous one. In fact it is used to move the stylet towards the back of the body. Thanks to the mechanism of the invention, the user carries out the same displacement movement of the arming button twice, these two movements having different effects.

This way of proceeding has the advantage of enabling the realization of a body of relatively small length and of ensuring a stroke of the arming button, which is compatible with the displacement of the user's finger, without obliging the user to change the position of his/her hand.

The cannula and the stylet are realised in such a way as to limit friction between the elements and thus allowing an optimal sliding of the stylet into the cannula.

The device of the invention avoids jamming of the stylet and the cannula, as well as the possible consequences of it, such as deformation or rupture.

By virtue of the geometry of the device, the elements which enable the guidance of the stylet and the cannula, as well as the propulsion and retaining elements for the stylet and the cannula are arranged symmetrically around a longitudinal axis materialized by the stylet. This ensures that there are few transversal forces. Such transversal forces have the effect of increasing the friction between the parts, of causing wear and of risks of rupture as well as of jamming. By suppressing these transversal forces, it is possible to use smaller springs as it is no longer necessary to fight against friction. The biopsy gun is thus easier to use since the arming is made easier. Moreover, the gun can be used more often since the jamming risk is reduced.

### BRIEF DESCRIPTION OF THE DRAWINGS

This invention and its advantages will be better understood with reference to the enclosed drawings and to the detailed description of a particular embodiment, in which:

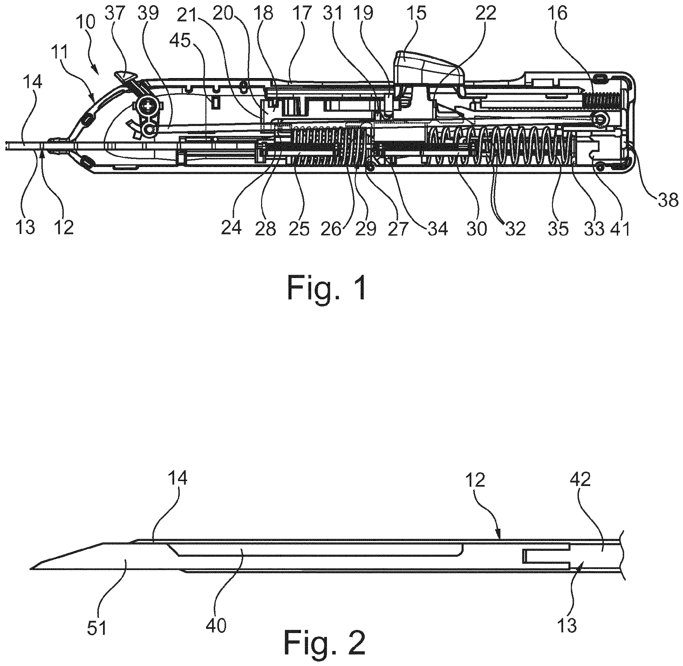

- Figure 1 is an overview of the device of the present invention; and
- Figure 2 is a cross section of a detail of the sampling device according to the invention.

### BEST MODE FOR CARRYING OUT THE INVENTION

With reference to the drawings, the sampling device 10 according to this invention essentially comprises a body 11 and a needle 12. The needle is formed by a stylet 13 and by a cannula 14. The stylet comprises a tip allowing a penetration of the needle into the organ from which one wishes to take a sample. Furthermore, this stylet comprises at least one notch 40. In practice, the stylet 13 comprises a relatively long notch that enables a sample of great length to be taken. The cannula 14 slides around the stylet 13 and is used both to section the tissue into which the stylet has penetrated and to keep in place the tissues taken at the time of the extraction of the needle from the organ.

The stylet is formed of two separate parts, namely a tip 51 for taking a sample and a rod 42 for the displacement of the tip. The sampling tip has a cross-sectional dimension so as to slide with a low clearance in the cannula.

According to an advantageous embodiment, the sampling tip 51 has a length superior to the length of the sampling notch 40, this length being between one and five times the notch length. This sampling tip 51 is advantageously made in a material having at the same time mechanical properties enabling this tip to penetrate a tissue from which a sample is to be taken, and properties enabling this tip to slide into the cannula. For instance this material may be a synthetic material.

The displacement rod 42 has a lower cross-sectional dimension compared to that of the cannula, even notably lower, so that a clearance between the rod and the cannula is present and that this rod can easily be displaced without friction into the cannula. However such displacement rod 42 must have a sufficiently large stiffness so as to largely transmit to the sampling tip 51 the push loads applied during a sampling shot on this rod, without any rod deformation. According to an advantageous embodiment, the displacement rod 42 is a metallic rod.

The body 11 essentially comprises an arming mechanism arranged to arm the needle 12 and a triggering device arranged to release a shot of the needle for the intended sampling. More particularly, the arming of the needle is carried out in two phases, namely a phase of arming the cannula 14 and a phase of arming the stylet 13.

The sampling is made by a shot of the needle. Such a shooting also comprises two phases, namely a displacement phase of the stylet 13 under the effect of a propelling power of the stylet, then a displacement phase of the cannula 14 under the effect of a propelling power of the cannula. Releasing a shot is achieved by liberating the displacement of the stylet. The displacement of the cannula is a consequence of the release of the stylet as it will be explained in detail below.

In practice, the mechanism for arming the cannula and the mechanism for arming the stylet use only one arming button 15 which acts differently depending on whether the arming of the cannula has already been carried out or not. This arming button cooperates with a return spring 16 of the arming button, this spring having the function to bring back the arming button 15 to the rest position i.e. towards the front of the body, when it is not manipulated.

The body of the device is formed by two parts which, once assembled, comprise guidance grooves intended to ensure the displacement of the parts. The body also comprises a slit 17 in which the arming button moves.

With reference to the figures, the arming button 15 cooperates with a platform 18. This platform can pivot around a platform axis 19 integral with the arming button. One of the ends of the platform, located near the front end of the sampling device i.e. the needle-end of the sampling device, comprises a widened zone 20, each end of this widened zone including a finger 21 whose function is described in detail below. The rear end of the platform comprises a pushing device 22 whose function is also described in detail below.

The platform 18 is connected to the arming button 15 by the platform axis 19 and by a return device (not represented) that can be in particular a spring or an elastic tab and which has the function of keeping this platform in a predefined position called a rest position.

The mechanism for arming the cannula 14 is intended to move the cannula into the shooting position. This cannula is coupled to a cannula slider 24. According to one advantageous embodiment, the cannula slider 24 comprises two fins 25 disposed in a plane containing also the cannula. These two fins 25 cooperate with two guide grooves realized in the body of the device so as to ensure an effective sliding motion of the cannula slider 24. This slider comprises, at its rear end, a retaining element 26 of the cannula slider. According to an advantageous embodiment, the retaining element is formed by two hooks. Advantageously, these hooks are symmetrical and realized so as to have a certain flexibility, which allows for them to be hooked onto a retaining device 27 of the cannula slider and to be unhooked from this device by approaching the hooks to each other. It is also possible to use only one hook or several hooks arranged asymmetrically.

Furthermore, the cannula slider 24 comprises a spur 28 cooperating with one of the fingers 21 of the platform. The cannula slider cooperates with a spring 29 for the propulsion of the cannula slider, which is arranged between the cannula slider 24 and the retaining device 27 of the cannula slider. This spring 29 is designed to supply the required force to propel the cannula slider towards the front of the body. The displacement of the cannula slider towards the back of the body effects the compression of this spring.

The mechanism for arming the stylet is intended for the displacement of the stylet 13 into the shooting position, this displacement being achieved after the cannula 14 has been armed. To that effect, the stylet 13 and more specifically the displacement rod 42 is kinematically coupled to a stylet slider 30. This stylet slider can comprise two parts, namely a support device and a guide device. The support device is integral with the stylet 13. According to a particular embodiment, it is overmolded on this stylet. The guide device comprises fins cooperating with guide grooves realized in the body 11 of the device. The guide device has a configuration such that the displacement of the guide device leads to the displacement of the support device. However, these two elements present a certain clearance between them. This clearance enables a relative displacement of the support device in comparison with the guide device in a plane substantially perpendicular to the needle. In principle, little or no clearance at all exists in a longitudinal axis in comparison with the needle. This clearance enables to take into account the manufacturing tolerances of the different elements of the device of the invention. The support device is in a "suspended" setup in comparison with the guide device.

This guiding device comprises a spur 31 near its front end and a retaining element 32 at its rear end. Like for the cannula slider, the retaining element 32 can be formed by two partially elastic hooks. It can also be formed only by one hook or by several hooks arranged symmetrically or asymmetrically.

This retaining element 32 can be hooked on a retaining device 33 of the stylet slider and can be unhooked from this device by approaching the hooks to each other.

Like for the cannula slider, the hooks of the stylet slider are sufficiently flexible to be able to be deformed one towards the other and sufficiently rigid to be able to be maintained on an adequate support.

The stylet slider 30 comprises, at its front end, i.e. at the side of the cannula slider, unlocking means 34 formed for instance by two inclined planes.

The guide device of the stylet slider cooperates with a spring 35 for the propulsion of the stylet slider, which is placed between the stylet slider 30 and the retaining device 33 of the stylet slider. This spring is designed to supply the required force to propel the stylet slider 30 towards the front of the body and to unlock the cannula slider. The displacement of the stylet slider towards the back of the body effects the compression of this spring.

In a way similar to the stylet slider, the cannula slider can also be formed of two parts, namely a support device of the cannula slider and a guide device of the cannula slider. Both parts can have a certain clearance between them so as to enable a relative displacement between the different parts. This further allows minimising the risks of jamming.

The device according to this invention further comprises a triggering device. According to an advantageous embodiment, this triggering device comprises two triggers 37, 38 connected together by a rod 39. One of the triggers 37 is placed in the front of the body, in front of the arming button 15 and the other trigger 38 is placed in the rear of the body. The rear trigger 38 is associated with a return spring of the trigger, designed to bring the trigger back in the original position after it has been pressed.

The rear trigger 38 comprises means for unlocking 41 the stylet slider formed by two elements arranged in inclined planes.

The sampling device according to this invention operates in the following way. Let us assume that the initial position is a position in which the cannula 14 and the stylet 13 are maximally extended towards the outside of the body 11 of the device. This position corresponds to the normal position of the device when it is not going to be used, i.e. rest position.

In a first phase, the arming of the cannula 14 is carried out. During this operation, the user actuates the arming button 15, making it slide towards the back of the device 10. The platform 18 being integral with the arming button 15, the displacement of the latter also draws the platform backwards. One of the fingers 21 of the platform 18 comes in contact with the spur 28 placed towards the front end of the cannula slider 24. The latter is thus displaced backwards, in opposition to the force of the spring 29 for the propulsion of the cannula slider. This movement is carried out until the retaining elements 26 of the cannula slider 24 enter into contact with the retaining device 27 of the cannula slider. This retaining device 27 is for instance a ring realized in the body of the device. The ring comprises a central hole in which the ends of the hooks of the cannula slider pass. These hooks lean on the back face of the ring and maintain the cannula slider 24 in opposition to the force of the propulsion spring of this cannula slider.

When the arming of the cannula is terminated, the arming button 15 is released. It returns to its initial position towards the front of the device, under the effect of the return spring 16 of the arming button.

During the forward displacement of the platform 18, following the forward displacement of the arming button 15, a ramp of the platform comes into contact with a plug 45 realized in the body. This ramp has the effect of rotating the platform 18 around the platform axis 19, against the force of the return device 23 of the platform. It should be noted that according to the chosen practical realization, it is also possible to provide for the return device of the platform to be constrained before the arming of the cannula and to be liberated when the arming of the cannula is terminated.

For the arming of the stylet 13, the arming button 15 is displaced backwards again. However, the platform 18 is no longer in the initial position. Indeed, the latter has pivoted around the platform axis 19, as the ramp of the platform has been displaced by the support against the plug 45. By this rotation, the finger 21 of the platform does not, on one side, come into contact with the spur 28 of the cannula slider and, on the other side, the pushing device 22 of the platform presses against the spur 31 of the guiding device of the stylet slider. Thus this slider is moved towards the back of the device, in opposition to the force of the spring 35 of propulsion of the stylet slider, until the retaining elements 32 of the support device of the stylet slider are arranged in the retaining device 33 of the stylet slider. This retaining device is similar to the retaining device 27 of the hooks of the cannula slider. It has thus advantageously an annular form with a hole in which the hooks of the stylet slider come to lie. It should be noted that the retaining element of the stylet slider could be realized on the guide device instead of being realized on the support device. Likewise, the index could rest on the support device in place of the guide device, as long as the displacement of the support device leads to the displacement of the guide device.

At this stage, the device is triggered out and ready for the shot. The device is stable in the sense that the cannula and stylet slider hooks are maintained against the corresponding retaining elements. The arming button 15 is released and returns to its initial position under the effect of the return spring of the arming button. The platform 18 also returns to its initial position.

When the needle is armed, the sampling is started by a shot. This shot can be started by means of one of the triggers 37, 38 which have the function of releasing the displacement of the stylet and the cannula by releasing the stylet slider 30. The stylet slider is first propelled towards the front of the body under the force of the spring 35 of stylet propulsion. During this propulsion, the fins of the guide device follow the guide grooves realized in the body of the device. The displacement of the guide device leads to the displacement of the support device. The displacement of the stylet slider involves the displacement of the displacement rod 42, which in turn involves the displacement of the sampling tip 51. This tip is guided by the cannula 14 almost without clearance in a plane perpendicular to the displacement direction of the needle, taking however into account the manufacturing tolerances of the different elements of the device of the invention. On the contrary, the displacement rod 42 presents a clearance with the cannula in such a way that there is no friction between the displacement rod 42 and the cannula 14.

The cannula slider 24 is then propelled towards the front of the device under the force of the spring 29 for cannula propulsion.

In the disclosed embodiment, the triggering mechanism comprises the two triggers and the rod 39 previously mentioned. The feature of having one of the triggers arranged in front of the body, in front of the tensioning button and the other arranged in the rear of the body enables the user to easily access the triggering mechanism, whatever is the position of the hand during the use of the device.

According to an advantageous embodiment, a security mechanism is provided for preventing a shot during an involuntary manipulation of one of the triggers and in particular of the front trigger. Before the release of the shot, it is necessary to laterally displace this front trigger 37 in relation to the body 11 in order to remove the security function of the mechanism. After the shot, it is necessary to laterally re-displace the front trigger 37 in order to reactivate the security function. This security is manual in the sense that the user has the choice of activating the function by displacing the trigger, or not activating it.

To release the shot, it is necessary to press one of the triggers 37, 38, the front or the rear one. Actually, in the disclosed embodiment, the shot is always released by a displacement of the rear trigger 38. However, the front trigger and the rear trigger being linked by the rod 39, a pressure on the front trigger has as result to move the rear trigger forward under the pressure of the rod. Thus the mechanism can be used by pressing either the rear trigger or the front trigger.

When the rear trigger 38 is pressed, the unlocking means 41 being part of the rear trigger (or means for unlocking the stylet slider) comes into contact with the hooks of the stylet slider and displaces them towards each other. In this way, they are released from the retaining device 33 of the stylet slider. This slider 30 is propelled forward under the effect of the propulsion spring 35 of the stylet slider.

The means 34 for unlocking the cannula slider comes into contact with the hooks of the cannula slider, presses these hooks towards the centre and releases the retaining elements 27 of the cannula slider. The cannula slider 24 advances under the effect of the propulsion spring 29 of the cannula. This slider advances until it arrives at a stop realized in the body of the device. At this stage, the shot is terminated and the device can be withdrawn from the organ from which samples have been taken.

After the arming of the stylet, the platform 18 has returned to its rest position under the effect of the return device of the platform. After the shot, the pieces of the device return to their initial positions. The sample taken is confined between the stylet 13 and the cannula 14, in the notch 23 of the sampling tip 51. This sample can be withdrawn by moving back the cannula, for instance by carrying out an arming movement as previously explained. When the arming of the cannula is terminated, it is possible to withdraw the sample. If a new sampling has to be carried out, the arming button is operated so as to arm the device totally and to make it ready for the shot. If it is not necessary to take a new sample, the arming is carried out as well and a blank shot is made.

The present invention has several advantages in comparison with the devices of the prior art. In particular, by the setup of the retaining elements 26, 32 of the stylet and cannula sliders, it is possible to provide at least two symmetrical hooks. The forces applied on these hooks to hold them by the retaining means as well as during their unhooking during a shot are symmetrical. On one hand, this ensures that there is no flexing and/or twist on the needle, and on the other hand, this enables a safer support of the hooks.

The sampling tip of the needle being relatively short with respect to the total length of the needle, the area in which there may be friction between the cannula and the stylet is also relatively short. As a result the risks of jamming are largely reduced. Moreover, since the necessary force to overcome friction is lower than in the devices of the prior art, it is possible to use springs having a lower stiffness while maintaining the same shooting speed and reducing the necessary force for the arming. It is also possible to use springs having the same stiffness i.e. that need the same force for the arming, while obtaining at the same time a higher shooting speed.

The clearance between the guide device and the support device forming the stylet slider also ensures an optimal displacement of the stylet in relation to the cannula and thus prevents the jamming or deformation of the needle.

In a similar way, the clearance between the guidance device of the cannula slider and the support device of the cannula slider also ensures an optimal displacement of the stylet with respect to the cannula and thus prevents the jamming or needle deformations.

According to an advantageous realization, the needle is off-center towards the bottom of the device 10. This enables the use of the device in an easier way with another apparatus as for example an echographic probe.

The device according to the invention can be operated by one single hand since the arming of the cannula and the arming of the stylet use the same arming button.

By the symmetrical construction of the retaining elements of cannula and stylet sliders and by the position of the propulsion springs of these sliders, the stresses are divided symmetrically around the axis of the needle. Thus, the risks of jamming between the stylet and the cannula are minimized, which in some implementations of the invention enables the use of the device several times and thus allows for a greater number of samples to be taken.

The reduction of the risks of jamming and the realisation of the stylet slider and/or the cannula slider in two elements that have a clearance among each other allows for the reduction of the force of the propulsion springs while maintaining a high displacement speed for the sliders. This is advantageous for the user because a smaller force is necessary for arming the device. The manipulation with a single hand is easier in this way.

Using guide grooves realized in the body of the device and slider fins moving in these grooves also ensures an optimal guidance and a diminution of the jamming risk.

## Claims

1. Sampling device (10) for taking at least one sample of soft tissue from an organ, said device (10) comprising a body (11) and a needle (12) formed by a stylet (13) and a cannula (14) coaxial with said stylet (13), said device (10) comprising a mechanism for arming the needle, designed for sequentially moving the cannula (14) and then the stylet (13) from a rest position wherein the stylet (13) and the cannula (14) are extended towards the outside of the body (11), to a shooting position wherein the stylet (13) and the cannula (14) are retracted towards the rear of the body (11), and a triggering mechanism designed to release the stylet (13) then the cannula (14) and to allow their displacement from the shooting position to the rest position, the cannula (14) being coupled kinematically to a cannula slider (24) comprising at least one retaining element (26) for retaining the cannula slider (24) in a shooting position, the stylet (13) being coupled kinematically to a stylet slider (30) comprising at least one retaining element (32) for retaining the stylet slider (30) in a shooting position and means (34) for unlocking the cannula slider (24), **characterized in** the stylet (13) being formed of two separate parts: a sampling tip (51) able to slide into the cannula (14) and a displacement rod (42) connecting said sampling tip (51) with the stylet slider (30), the displacement rod (42) having a lower cross-sectional dimension compared to that of the cannula (14), so that a clearance between the displacement rod (42) and the cannula (14) is present and that the displacement rod (42) can easily be displaced without friction into the cannula (14).

2. Sampling device (10) according to claim 1, **characterized in that** the displacement rod (42) has a cross-sectional dimension such that it allows the transmission of movement from the stylet slider (30) to the sampling tip (51).

3. Sampling device (10) according to claim 1, **characterized in that** the sampling tip (51) has a length between one and five times the length of a sampling notch (40) formed in the stylet (13).

4. Sampling device (10) according to claim 1, **characterized in that** the sampling tip (51) is made of a synthetic material.

5. Sampling device (10) according to claim 1, **characterized in that** the displacement rod (42) is made of metal.

6. Sampling device (10) according to claim 1, wherein the mechanism for arming the needle (12) comprises only one arming button (15).

7. Sampling device (10) according to claim 6, wherein the arming button (15) cooperates with a return spring (16) configured to bring back the arming button (15) to the rest position when it is not manipulated.

8. Sampling device (10) according to claim 7, wherein the body (11) comprises a slit (17) in which the arming button (15) moves.

9. Sampling device (10) according to claim 1, wherein the retaining element (26) for retaining the cannula slider (24) is formed by two symmetrical, flexible hooks adapted to hook onto a retaining device (27) of the cannula slider (24) and to be unhooked from the retaining device (27) by approaching the hooks to each other.

10. Sampling device (10) according to claim 1, wherein the needle (12) is off-center towards the bottom of the device (10).

## Patentansprüche

1. Probenahmevorrichtung (10) zum Nehmen wenigstens einer Weichgewebeprobe von einem Organ, wobei die Vorrichtung (10) einen Körper (11) und eine Nadel (12), die von einem Stilett (13) und einer mit dem Stilett (13) koaxialen Kanüle (14) gebildet wird, umfasst, wobei die Vorrichtung (10) einen Mechanismus zum Scharfstellen der Nadel umfasst, der dafür ausgelegt ist, nacheinander die Kanüle (14) und dann das Stilett (13) aus einer Ruhestellung, bei der das Stilett (13) und die Kanüle (14) zu der Außenseite des Körpers (11) ausgefahren sind, zu einer Abfeuerstellung, bei der das Stilett (13) und die Kanüle (14) zu der Rückseite des Körpers (11) zurückgezogen sind, zu bewegen, und einen Auslösemechanismus, der dafür ausgelegt ist, das Stilett (13) und dann die Kanüle (14) freizugeben und ihre Verschiebung von der Abfeuerstellung in die Ruhestellung zu erlauben, wobei die Kanüle (14) kinematisch an einen Kanülenschieber (24) gekoppelt ist, der wenigstens ein Halteelement (26) zum Halten des Kanülenschiebers (24) in einer Abfeuerstellung umfasst, wobei das Stilett (13) kinematisch an einen Stilettschieber (30) gekoppelt ist, der wenigstens ein Halteelement (32) zum Halten des Stilettschiebers (30) in einer Abfeuerstellung und eine Einrichtung (34) zum Entriegeln des Kanülenschiebers (24) umfasst, **dadurch gekennzeichnet, dass** das Stilett (13) aus zwei getrennten Teilen besteht: einer Probenahmespitze (51), die fähig ist, in die Kanüle (14) zu gleiten, und einem Verstellstab (42), der die Probenahmespitze (51) mit dem Stilettschieber (30) verbindet, wobei der Verstellstab (42) eine kleinere Querschnittsabmessung als die Kanüle (14) aufweist, so dass ein Abstand zwischen dem Verstellstab (42) und der Kanüle (14) vorliegt und der Verstellstab (42) leicht und ohne Reibung in die Kanüle (14) verschoben werden kann.

2. Probenahmevorrichtung (10) gemäß Anspruch 1, **dadurch gekennzeichnet, dass** der Verstellstab (42) eine Querschnittsabmessung aufweist, die eine Bewegungsübertragung von dem Stilettschieber (30) zu der Probenahmespitze (51) erlaubt.

3. Probenahmevorrichtung (10) gemäß Anspruch 1, **dadurch gekennzeichnet, dass** die Probenahmespitze (51) eine Länge zwischen ein- und fünfmal der Länge einer in dem Stilett (13) gebildeten Probenahmevertiefung (40) aufweist.

4. Probenahmevorrichtung (10) gemäß Anspruch 1, **dadurch gekennzeichnet, dass** die Probenahmespitze (51) aus einem synthetischen Material besteht.

5. Probenahmevorrichtung (10) gemäß Anspruch 1, **dadurch gekennzeichnet, dass** der Verstellstab (42) aus Metall besteht.

6. Probenahmevorrichtung (10) gemäß Anspruch 1, wobei der Mechanismus zum Scharfstellen der Nadel (12) nur einen Scharfstellknopf (15) umfasst.

7. Probenahmevorrichtung (10) gemäß Anspruch 6, wobei der Scharfstellknopf (15) mit einer Rückstellfeder (16) zusammenwirkt, die dafür gestaltet ist, den Scharfstellknopf (15) in die Ruhestellung zurückzubringen, wenn er nicht betätigt wird.

8. Probenahmevorrichtung (10) gemäß Anspruch 7, wobei der Körper (11) einen Schlitz (17) umfasst, in dem sich der Scharfstellknopf (15) bewegt.

9. Probenahmevorrichtung (10) gemäß Anspruch 1, wobei das Halteelement (26) zum Halten des Kanülenschiebers (24) aus zwei symmetrischen flexiblen Haken gebildet wird, die dafür ausgelegt sind, in eine Haltevorrichtung (27) des Kanülenschiebers (24) einzuhaken und durch Annähern der Haken aneinander aus der Haltevorrichtung (27) auszuhaken.

10. Probenahmevorrichtung (10) gemäß Anspruch 1, wobei die Nadel (12) in Richtung zu dem Boden der Vorrichtung (10) exzentrisch ist.

## Revendications

1. Dispositif d'échantillonnage (10) pour le prélèvement d'au moins un échantillon de tissu mou sur un organe, ledit dispositif (10) comprenant un corps (11) et une aiguille (12) formée par un stylet (13) et une canule (14) coaxiale audit stylet (13), ledit dispositif (10) comprenant un mécanisme destiné à armer l'aiguille, conçu pour déplacer séquentiellement la canule (14) puis le stylet (13) d'une position de repos dans laquelle le stylet (13) et la canule (14) sont étendus vers l'extérieur du corps (11), à une position de tir dans laquelle le stylet (13) et la canule (14) sont rétractés vers l'arrière du corps (11), et un mécanisme de déclenchement conçu pour libérer le stylet (13) puis la canule (14) et pour pour permettre leur déplacement de la position de tir à la position de repos, la canule (14) étant accouplée cinématiquement à un coulisseau (24) de canule comportant au moins un élément de retenue (26) destiné à retenir le coulisseau (24) de canule dans une position de tir, le stylet (13) étant accouplé cinématiquement à un coulisseau (30) de stylet comportant au moins un élément de retenue (32) destiné à retenir le coulisseau (30) de stylet dans une position de tir et des moyens (34) destinés à déverrouiller le coulisseau (24) de canule, **caractérisé en ce que** le stylet (13) est formé de deux parties distinctes : une pointe d'échantillonnage (51) pouvant coulisser dans la canule (14) et une tige de déplacement (42) reliant ladite pointe d'échantillonnage (51) au coulisseau (30) de stylet, la tige de déplacement (42) ayant une dimension en coupe transversale inférieure par rapport à celle de la canule (14), de sorte qu'un débattement entre la tige de déplacement (42) et la canule (14) existe et que la tige de déplacement (42) puisse facilement être déplacée sans frottement dans la canule (14).

2. Dispositif d'échantillonnage (10) selon la revendication 1, **caractérisé en ce que** la tige de déplacement (42) a une dimension en coupe transversale telle qu'elle permet la transmission d'un mouvement depuis le coulisseau (30) de stylet vers la pointe d'échantillonnage (51).

3. Dispositif d'échantillonnage (10) selon la revendication 1, **caractérisé en ce que** la pointe d'échantillonnage (51) a une longueur entre une et cinq fois la longueur d'une encoche d'échantillonnage (40) formée dans le stylet (13).

4. Dispositif d'échantillonnage (10) selon la revendication 1, **caractérisé en ce que** la pointe d'échantillonnage (51) est en matériau synthétique.

5. Dispositif d'échantillonnage (10) selon la revendication 1, **caractérisé en ce que** la tige de déplacement (42) est métallique.

6. Dispositif d'échantillonnage (10) selon la revendication 1, dans lequel le mécanisme d'armement l'aiguille (12) comprend un seul bouton d'armement (15).

7. Dispositif d'échantillonnage (10) selon la revendication 6, dans lequel le bouton d'armement (15) coopère avec un ressort de rappel (16) conçu pour ramener le bouton d'armement (15) à la position de repos lorsqu'il n'est pas manipulé.

8. Dispositif d'échantillonnage (10) selon la revendication 7, dans lequel le corps (11) comprend une fente (17) dans laquelle se déplace le bouton d'armement (15).

9. Dispositif d'échantillonnage (10) selon la revendication 1, dans lequel l'élément de retenue (26) pour retenir le coulisseau (24) de canule est formé par deux crochets symétriques souples conçus pour s'accrocher à un dispositif de retenue (27) du coulisseau (24) de canule et pour être décrochés du dispositif de retenue (27) en rapprochant les crochets l'un de l'autre.

10. Dispositif d'échantillonnage (10) selon la revendication 1, dans lequel l'aiguille (12) est excentrée vers la partie inférieure du dispositif (10).
